# EUROPEAN PATENT APPLICATION

(11) **EP 3 460 444 A1**
(43) Date of publication of application: **27.03.2019**
(21) Application number: 16902542.6
(22) Date of filing: 27.12.2016
(51) Int. Cl.: G01N 1/14, G01N 1/40, G01N 15/14, G01N 33/483

(54) **HIV DIAGNOSTIC METHOD USING CD4 AND CD8 CELL INFORMATION**

(30) Priority: 16.05.2016 KR 20160059616
(71) Applicant: Glory Biotech Corp., Seoul 07995 (KR)
(72) Inventor: SUNG, Yeon-Moon, Seoul 07987 (KR); LIM, Taekyu, Seoul 07983 (KR)
(74) Representative: EP&C
(86) International application number: PCT/KR2016/015300
(87) International publication number: WO 2017/200175

(57) **Abstract**

A method of diagnosing HIV is provided. The method of diagnosing HIV according to an embodiment of the present invention is a method of diagnosing HIV using an HIV diagnostic apparatus comprising a nanofilter, wherein the nanofilter comprises an upper body (100) for collecting cells, the upper body (100) has a plurality of filtration holes (120) formed through a substrate (110), any one filtration hole of the plurality of filtration holes (120) is adjacent to other filtration holes, and an interval between a center of any one filtration hole and those of the other filtration holes adjacent thereto is 23 µm to 27 µm. The method of diagnosing HIV comprises (a) collecting leukocytes, among the cells, using the nanofilter, (b) radiating light toward the entire area of the nanofilter using a light source unit (500), (c) sensing the light passing through the nanofilter using a light-sensing unit (600), (d) outputting an image of the entire area of the nanofilter through a display means (700) based on information obtained by the light-sensing unit (600), and (e) analyzing the image outputted through the display means (700) using an analyzing means (800). The step (e) may comprise analyzing information on the ratio of a CD4 positive T cell count to a CD8 positive T cell count using the analyzing means (800).

## Description

### Technical Field

The present invention relates to a method of diagnosing HIV using information on CD4 and CD8 cells, and more particularly to a diagnostic method of determining starting for AIDS treatment and effectively monitoring an AIDS treatment process by collecting cells using a nanofilter and observing CD4 positive T cells and CD8 positive T cells that are collected.

### Background Art

The number of persons infected with AIDS worldwide is expected to reach 40 million, and most of the infected persons are emerging, mostly in developing countries. HIV, which is the virus causative of AIDS, infects CD4 positive T cells in the blood and gradually destroys the CD4 positive T cells over a latency period of several months to 10 years, thereby lowering the immune function of patients. The patients exhibiting such impaired immunity may die due to various complications.

There is a method of measuring CD4 positive T cells in the blood in order to decide whether AIDS treatment is necessary. Examples of the measurement method comprise a method of counting the absolute number of CD4 positive T cells, a method of calculating the ratio of a CD4 positive T cell count in the total lymphocyte count (CD4/lymphocyte), and a method of calculating the ratio of a CD4 positive T cell count to a CD8 positive T cell count (CD4/CD8). Since the variation of CD4/lymphocyte and CD4/CD8 values is relatively small regardless of ages, the methods of calculating CD4/lymphocyte and CD4/CD8 ratios are mainly used. In the WHO guidelines, the time point at which a CD4 positive T cell count is 350 cells/µl or less, CD4/lymphocyte is 0.25 or less in the case of children younger than 5 years, or CD4/CD8 is less than 1 is considered to be the starting time point for AIDS treatment.

The method of measuring the CD4 positive T cell count as described above is typically performed using a flow cytometry method. The flow cytometry method has the advantage of high accuracy, but it is difficult to use the flow cytometry method in developing countries because the method requires trained workers and the equipment therefor is expensive.

The related conventional technology will be described as follows.

U.S. Patent Application Publication No. 2014-0273018 discloses a device and a system for counting the absolute number of CD4 positive T cells and calculating CD4/lymphocyte and CD8/lymphocyte ratios in order to decide whether the risk of developing AIDS exists. However, since the intervals between the holes of the nanofilter for collecting leukocytes are random, reproducibility cannot be obtained in an antigen-antibody reaction. Further, since nonspecific antibodies are not easily washed out, it is not easy to observe results.

Korean Patent Application Publication No. 2012-0042518 discloses a metal screen filter for collecting cancer cells. However, the patent is for the purpose of recovering cancer cells after the cancer cells are collected, and has an object different from that of the present invention.

### Disclosure

### Technical Problem

The present invention relates to a diagnostic method of determining starting for AIDS treatment and effectively monitoring an AIDS treatment process while filtering target cells with high efficiency using a nanofilter.

### Technical Solution

In order to accomplish the above object, an embodiment of the present invention provides a method of diagnosing HIV using an HIV diagnostic apparatus comprising a nanofilter. The nanofilter comprises an upper body (100) for collecting cells, the upper body (100) has a plurality of filtration holes (120) formed through a substrate (110), any one filtration hole of the plurality of filtration holes (120) is adjacent to other filtration holes, and an interval between a center of any one filtration hole and centers of the other filtration holes adjacent thereto is 23 µm to 27 µm. The method of diagnosing HIV comprises (a) collecting leukocytes among the cells using the nanofilter, (b) radiating light toward the entire area of the nanofilter using a light source unit (500), (c) sensing the light passing through the nanofilter using a light-sensing unit (600), (d) outputting an image of the entire area of the nanofilter through a display means (700) based on information obtained using the light-sensing unit (600), and (e) analyzing the image outputted through the display means (700) using an analyzing means (800). (e) The analyzing the image comprises analyzing information on a ratio of a CD4 positive T cell count to a CD8 positive T cell count using the analyzing means (800).

In the embodiment, preferably, (e) the analyzing the image further comprises analyzing information on the CD4 positive T cell count and a ratio of a CD4 positive cell count to a lymphocyte cell count using the analyzing means 800, and determining a time point at which the information analyzed using the analyzing means (800) indicates that the CD4 positive T cell count is less than 350 cells/µl, or the ratio of the CD4 positive T cell count to the CD8 positive T cell count is less than 1, or the ratio of the CD4 positive T cell count to the lymphocyte cell count is 0.25 or less as a starting time point for AIDS treatment.

In the embodiment, preferably, planes of the filtration holes (120) have a circular shape, and diameters of the filtration holes (120) are 3 µm to 5 µm.

In the embodiment, preferably, the substrate (110) is made up of a metal material or a plastic material.

In the embodiment, preferably, the nanofilter further comprises a filtration unit (130), which is positioned on an upper side of the substrate (110) and to which a sample solution is added, a suction unit (140), which is positioned on a lower side of the substrate (110) and to which a material not filtered through the filtration holes (120) is moved, and a flow channel (300), through which the suction unit (140) and a lower body (400) are connected to each other, and the method further comprise, before (a) the collecting the leukocytes, separating the lower body (400) from the upper body (100) at a constant rate, so that the sample solution added to the filtration unit (130) is sucked into the suction unit (140) at a predetermined rate.

In the embodiment, preferably, the predetermined rate is 100 µl/min to 500 µl/min.

In the embodiment, preferably, the light-sensing unit (600) comprises a lens (610) through which the light passing through the nanofilter is converted to a parallel light form, an excitation light filter (620) through which light having a specific wavelength among light components passing through the lens (610) passes, and an image sensor (630) sensing the light passing through the excitation light filter (620).

In the embodiment, preferably, the method further comprises, after (e) the analyzing the image, displaying the information analyzed using the analyzing means (800) through the display means (700).

In the embodiment, preferably, the cells further comprise JM cells or IMMUNO-TROL cells.

In the embodiment, preferably, the method further comprises, before (a) the collecting the leukocytes, reacting a CD4 positive T cell and a CD8 positive T cell with Alexa Fluor 488 marked anti-Human CD4 IgG antibody and PE-Alexa Fluor 610 marked anti-Human CD8 IgG, respectively, for 10 minutes to 30 minutes.

### Advantageous Effects

According to the present invention, the interval between the center of any one of the filtration holes and those of other filtration holes adjacent thereto is constant, and it is possible to collect target cells with high efficiency by adjusting the size of the filtration hole for the cell collection.

Further, since a nanofilter according to the present invention comprises a substrate of a metal material or a plastic material, it is possible to easily wash out an antibody that is not reacted with an antigen.

Further, since a sample solution is sucked at a constant rate, it is possible to collect the target cells with high efficiency.

Further, it is possible to observe the image of the entire area of the nanofilter in real time while collecting the target cells.

Further, since CD4/lymphocyte and CD4/CD8 ratios can be calculated in a diagnostic method using an analysis apparatus according to the present invention, it is possible to determine starting for AIDS treatment.

Further, it is possible to observe CD4 positive T cells and CD8 positive T cells and also observe CD antigens, such as CD19 and CD45, which are present in leukocytes.

### Description of Drawings

FIG. 1A is a perspective view of a nanofilter according to an embodiment of the present invention;
FIG. 1B shows that lower body is separated from the upper body in the nanofilter of FIG.1A and the sample solution added to the filtration unit is sucked;
FIG. 1C is a cross-sectional view of the nanofilter of FIG. 1B, taken along line A-A';
FIG. 2A is a schematic view of an analysis apparatus using the nanofilter of FIG. 1A;
FIG. 2B is a photomicrograph of the nanofilter of FIG. 1A;
FIG. 3A shows a fluorescence microscope image observed after dyeing leukocytes collected using the nanofilter of FIG. 1A;
FIG. 3B shows the results of observation of CD4 positive T cells and CD8 positive T cells using an analysis apparatus according to the embodiment of the present invention, and it was found that (1) CD8 positive T cells labeled with PE and (2) CD4 positive T cells labeled Alexa Flur 488;
FIG. 4A is a graph showing the correlation between CD4/CD8 ratios measured using a fluorescence microscope and the analysis apparatus according to the embodiment of the present invention, and FIG. 4B is a graph showing the CD4/CD8 ratio using the analysis apparatus according to the embodiment of the present invention;
FIG. 5 is a graph of the recovery rate of leukocytes according to the size of a filtration hole;
FIG. 6 is a graph of the recovery rate of leukocytes according to the suction rate of the sample solution;
FIG. 7 shows the results of comparison of measured values with those of a flow cytometry method, which is a conventional method for calculating a CD4/CD8 ratio, etc.; and

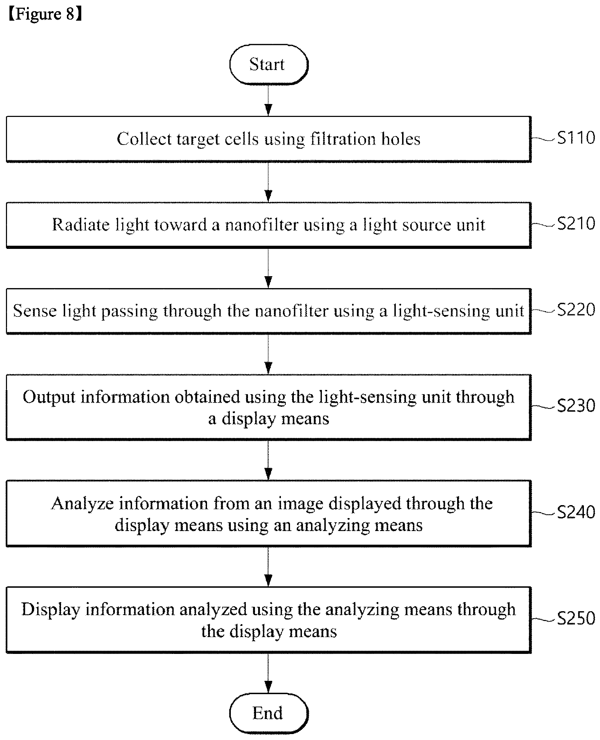

FIG. 8 shows a flowchart of a diagnostic method using the analysis apparatus of the present invention.

### Best Mode

### 1. Description of nanofilter

Hereinafter, a nanofilter according to an embodiment of the present invention will be described.

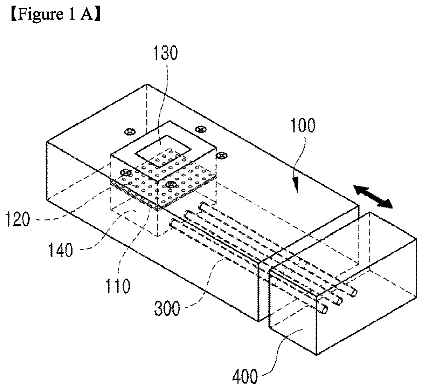

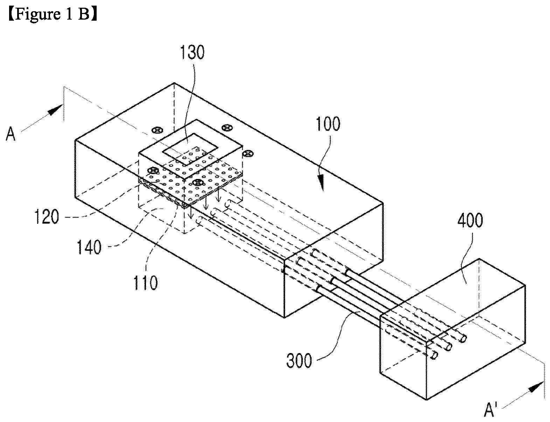

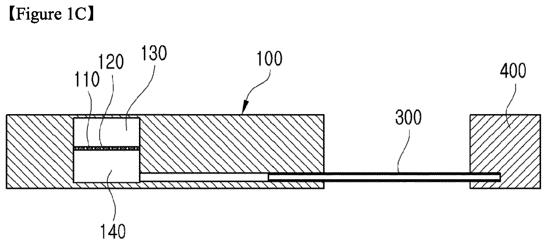

Referring to FIGS. 1A and 1B, the nanofilter according to the embodiment of the present invention comprises an upper body (100) for collecting cells.

The upper body (100) has a plurality of filtration holes (120) formed through a substrate (110) therein so that target cells (for example, leukocytes) are collected using the plurality of filtration holes (120). Erythrocytes that are present in the blood may also be collected when the leukocytes are collected. However, erythrocytes are not filtered in the filtration holes (120) because the size of the erythrocytes is reduced through an erythrocyte lysis process before the blood is added to a filtration unit (130). Therefore, the leukocytes that need to be collected may be collected using the filtration holes (120). A detailed description of the erythrocyte lysis process will be omitted.

The size of each of the plurality of filtration holes (120) may be adjusted to thus collect the target cells. When the filtration holes (120) are formed so as to be smaller than the target cells, the target cells do not pass through the filtration holes (120), and thus the desired target cells may be collected. The interval between the center of any one filtration hole and those of other filtration holes adjacent thereto is preferably 23 µm to 27 µm. When the separation distance is too short, the images of the filtration holes (120) may be seen overlapped in the image displayed through a display means (700) which will be described later. When the distance is too long, the number of filtration holes (120) for collecting the target cells may not be sufficient.

Preferably, the planes of the plurality of filtration holes (120) have a circular shape, and the diameters of the filtration holes (120) are 3 µm to 5 µm.

When the diameter of the filtration hole (120) is larger than the above-described range, since the leukocytes cannot be sufficiently collected, the recovery rate of the leukocytes contained in the sample blood is reduced. Details of the experiment on the recovery rate of the leukocytes according to the size of the diameter of the filtration hole (120) will be described later.

The substrate (110) preferably is made up of a metal material or a plastic material. The substrate (110) is made up of the metal material or the plastic material, whereby, after the antigen-antibody reaction of the antigen of the target cell and a fluorescently labeled antibody, the antibody that is not reacted with the antigen may be easily washed out (washing process).

The nanofilter according to the embodiment of the present invention further comprises a filtration unit (130), which is connected to the substrate (110) and is positioned on the upper side of the substrate (110), and a suction unit (140), which is connected to the substrate (110) and is positioned on the lower side of the substrate (110).

The filtration unit (130) is a room to which the sample solution containing the target cells is added. Since the filtration unit (130) comprises a room carrying a predetermined capacity, the sample solution added to the filtration unit (130) is filtered using the filtration holes (120), and materials that are not filtered are moved to the suction unit (140).

The suction unit (140) is a room to which the materials that are not filtered using the filtration holes (120) are moved. Since materials having a size smaller than that of the filtration hole (120) are not filtered by the filtration hole (120), the materials move through the filtration holes (120) to the suction unit (140).

The nanofilter according to the embodiment of the present invention further comprises a flow channel (300) through which the suction unit (140) and a lower body (400) are connected to each other.

The lower body (400) is separated from the upper body (100) at a constant rate to thus serve as a pump for sucking the sample solution. Referring to FIG. 1B, the lower body (400) is separated from the upper body to thus suck air from the filtration unit (130), whereby the sample solution added to the filtration unit (130) is sucked. When the sample solution is not sucked, the sample solution in the filtration unit (130) is not filtered but remains in the filtration unit (130). The sample solution is sucked so as to be filtered, which contrasts with the case of the nanofilter where there is no suction of the sample solution.

Since the lower body (400) is separated from the upper body (100) at a constant rate, the sample solution is sucked at a predetermined rate, and the predetermined rate is preferably 100 µl/min to 500 µl/min.

The case where the suction rate is slow is not different from the case of the nanofilter where there is no suction. In the case where the suction rate is fast, the leukocytes contained in the sample solution shrink to thus pass through the filtration holes (120), so that the recovery rate of the leukocytes is reduced. Details of the experiment on the recovery rate of the leukocytes according to the suction rate will be described later.

### 2. Description of analysis apparatus using nanofilter

Hereinafter, an analysis apparatus using a nanofilter according to the embodiment of the present invention will be described.

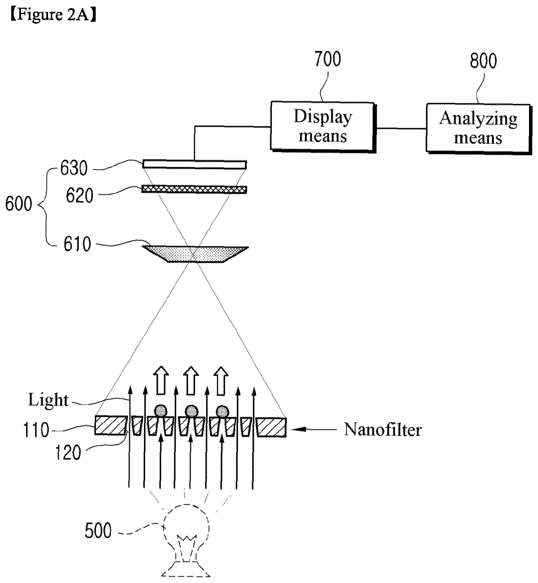

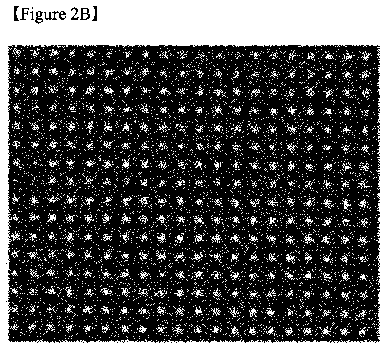

Referring to FIG. 2A, the analysis apparatus according to the embodiment of the present invention comprises the nanofilter according to the embodiment of the present invention, a light source unit (500) which is positioned on the lower side of the nanofilter and which radiates light toward the nanofilter, and a light-sensing unit (600) which is disposed so as to face the light source unit (500) while the nanofilter is interposed there between and which senses light passing through the nanofilter.

In the nanofilter, target cells (for example, leukocytes) are collected using the upper body (100). Since the method for collecting the target cells has been described above, a detailed description thereof will be omitted.

The light source unit (500) radiates light toward the nanofilter. The target cells collected by the nanofilter are bound to the fluorescently labeled antibody. A fluorescent material absorbs light having a specific wavelength and thus is in an excited state. Since white light comprises light of all wavelengths in a visible-ray region, it may excite the fluorescent material regardless of the type of fluorescent material. Therefore, the light source unit (500) is preferably an LED, a mercury lamp, or a halogen lamp that radiates white light.

The light-sensing unit (600) senses light passing through the nanofilter. The light-sensing unit (600) comprises a lens (610) positioned at a lower portion thereof, an excitation light filter (620) positioned on the upper side of the lens (610), and an image sensor (630) positioned on the upper side of the excitation light filter (620) .

The lens (610) serves to convert light passing through the nanofilter into a parallel light form. The lens (610) is preferably an optical part that makes the light parallel. The parallel light form of light reaches the image sensor (630) by converting light into the parallel light form, thereby facilitating image observation.

Only light having a specific wavelength passes through the excitation light filter (620). The fluorescent material with which the antibody is labeled absorbs specific wavelength of light radiated by the light source unit (500), and then is in an excited state. The state of the fluorescent material returns to a ground state to thus emit the wavelength of specific region. The excitation light filter (620) allows only the wavelength of the specific region to pass therethrough and blocks the other wavelength so that only fluorescently labeled target cells are observed.

The image sensor (630) serves to sense light passing through the excitation light filter (620) and then convert the light into an electric signal, thus forming a digital image. Accordingly, the light passing through the nanofilter is converted into the digital image. The image sensor (630) is preferably a CMOS sensor.

The analysis apparatus according to the embodiment of the present invention further comprises a display means (700) for outputting information obtained using the light-sensing unit (600) and an analyzing means (800) for analyzing information from the image displayed through the display means (700).

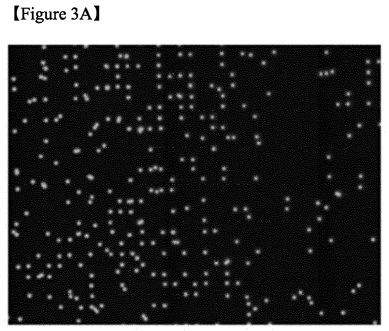

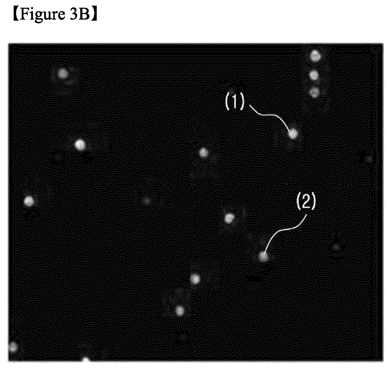

The display means (700) displays the digital image converted by the image sensor (630). This makes it possible to visually confirm fluorescently labeled target cells (see FIGS. 3A and 3B).

The analyzing means (800) serves to analyze the image displayed through the display means (700). The information that is analyzed may be a CD4/lymphocyte ratio or a CD4/CD8 ratio.

The display means (700) further displays information analyzed using the analyzing means (800). This allows visual confirmation of the CD4/lymphocyte ratio or the CD4/CD8 ratio.

The display means (700) comprises a notification unit, and the notification unit is operated when the information analyzed using the analyzing means (800) indicates that a CD4 positive T cell count is less than 350 cells/µl or that the CD4/CD8 ratio is less than 1. Therefore, it is possible to audibly confirm whether the risk of developing AIDS exists.

### 3. Description of diagnostic method using analysis apparatus

Hereinafter, a diagnostic method using an analysis apparatus according to the embodiment of the present invention will be described.

The diagnostic method according to the embodiment of the present invention comprises (a) collecting leukocytes among target cells using a nanofilter, (b) radiating light toward the entire area of the nanofilter using a light source unit (500), (c) sensing the light passing through the nanofilter using a light-sensing unit (600), (d) outputting an image of the entire area of the nanofilter through a display means (700) based on information obtained using the light-sensing unit (600), and (e) analyzing the image outputted through the display means (700) using an analyzing means (800). The step (e) comprises analyzing information on the ratio of a CD4 positive T cell count to a CD8 positive T cell count using the analyzing means (800).

The step (a) is a step of collecting the target cells using the nanofilter. The target cells are reacted with a fluorescently labeled antibody before a sample solution containing the target cells is added to an upper body (100). The target cells are preferably leukocytes, and one or more types of the antibodies may be reacted with the leukocytes. Through this, various types of target cells can be visually observed at the same time.

The step (b) is a step of radiating light toward the entire area of the nanofilter using the light source unit (500). The fluorescent material with which the antibody is labeled absorbs light of a specific wavelength and becomes an excited state.

The step (c) is a step of sensing light passing through the nanofilter using the light-sensing unit (600) (S220). The light-sensing unit (600) comprises a lens (610), an excitation light filter (620), and an image sensor (630) .

The lens (610) facilitates image observation by converting the light passing through the nanofilter into a parallel light form. The parallel light form of light passing through the lens (610) passes through the excitation light filter (620).

The excitation light filter (620) allows only the wavelength of specific region to pass therethrough. The state of the fluorescent material that has absorbed the light radiated from the light source unit (500) returns from an excited state to a ground state to thus emit the wavelength of specific region. The excitation light filter (620) changes the region of the wavelength to be passed according to the type of the fluorescent material so that only fluorescently labeled target cells are observed.

The image sensor (630) serves to sense light passing through the excitation light filter (620) and then convert the light into an electric signal, thus forming a digital image. Accordingly, the light passing through the nanofilter is converted into the digital image.

The step (d) is a step of outputting the image of the entire area of the nanofilter through the display means (700) based on information obtained using the light-sensing unit (600). The digital image converted by the light-sensing unit (600) is capable of being visually confirmed using the display means (700). When various types of antibodies are used during the step (a), images of different colors may be simultaneously checked according to the type of the fluorescent material with which the antibody is labeled.

The step (e) is a step of analyzing the image outputted through the display means (700) using the analyzing means (800). For example, when the anti-CD4 antibody (green fluorescent material is labeled) and the anti-CD8 antibody (yellow fluorescent material is labeled) are used during the step (a), information on the ratio of the CD4 positive T cell count to the CD8 positive T cell count may be analyzed using the image displayed through the display means (700).

In the step (e), the analyzing means (800) may serve to analyze not only the information on the ratio of the CD4 positive T cell count to the CD8 positive T cell count, but also information on the CD4 positive T cell count and the ratio of a lymphocyte count to the CD4 positive T cell count.

Further, the diagnostic method according to the embodiment of the present invention further comprises determining a time point at which the information analyzed using the analyzing means (800) indicates that the CD4 positive T cell count is less than 350 cells/µl, or the ratio of the CD4 positive T cell count to the CD8 positive T cell count is less than 1, or the ratio of the CD4 positive T cell count to the lymphocyte cell count is 0.25 or less as a starting time point for AIDS treatment. Accordingly, the starting time point for AIDS treatment may be simply and accurately determined using the diagnostic method according to the embodiment of the present invention.

### 4. Experiment on the recovery rate of leukocytes according to the size of filtration holes

An experiment was performed in order to confirm the recovery rate of leukocytes according to the size of the filtration holes (120) of the nanofilter according to the embodiment of the present invention.

The blood sample added to the filtration unit (130) was sucked at a rate of 200 µl/min, and the experiment was performed using five blood samples.

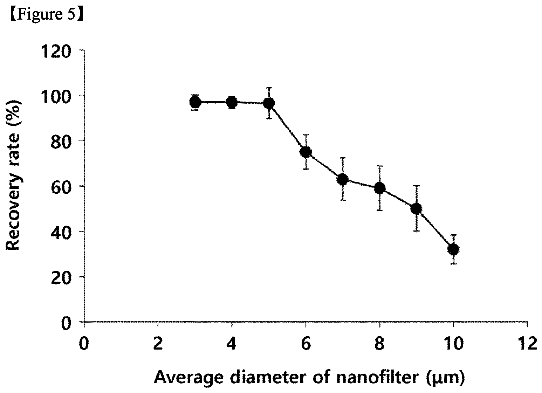

As shown in FIG. 5, when the diameter of the filtration hole (120) is 3 µm to 5 µm, it can be found that the recovery rate of leukocytes is high. However, when the diameter of the filtration hole (120) is 6 µm, it can be found that the recovery rate of the leukocytes is drastically reduced. Based on these results, it was found that the diameter of the filtration hole (120) is preferably 3 µm to 5 µm.

### 5. Experiment on the recovery rate of leukocytes according to the suction rate of a sample solution

An experiment was performed in order to find the recovery rate of leukocytes according to the suction rate of a sample solution using the nanofilter according to the embodiment of the present invention.

The blood samples added to the filtration unit (130) were sucked at different rates respectively, the diameter of the filtration hole (120) was fixed to 3 µm, and the experiment was performed using five blood samples.

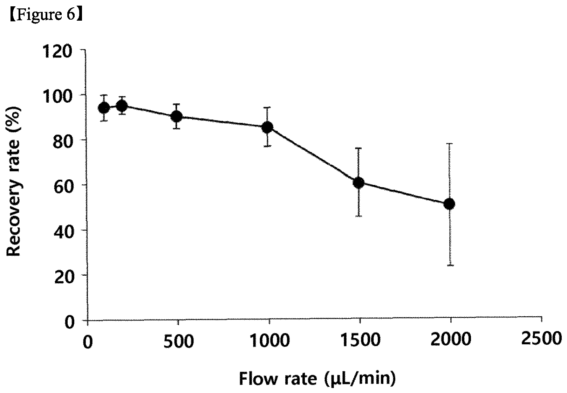

As shown in FIG. 6, when the suction rate is 100 µl/min to 500 µl/min, it can be found that the recovery rate of the leukocytes is high. However, when the suction rate exceeds the above-described range, it can be found that the recovery rate of the leukocytes is reduced. Based on these results, it was found that the suction rate of the sample solution is preferably 100 µl/min to 500 µl/min.

### 6. Verification experiment 1

An experiment was performed in order to find the superiority of the efficiency of collection of target cells using the analysis apparatus according to the embodiment of the present invention.

JM cells, which are cultured cell strains derived from T lymphoma, were used to perform collection using the analysis apparatus of FIG. 2A. The cultured JM cells were stained and washed, and then immune cells were filtered using a cell strainer (40 µm, BD Biosciences). After that, a cell count was measured using a hemocytometer (Fuchs-Rosenthal), and a cell sample (5 × 10⁴ cells/ml) was prepared. After the suction unit (140) of the nanofilter was filled with PBS, 200 µl of the fluorescently labeled JM cell sample that was prepared was added to the filtration unit (130), and the sample added to the nanofilter was sucked at 200 µl/min, thus performing cell collection using the nanofilter. After the collection, the cells filtered by the nanofilter were fixed for 15 minutes using 4% paraformaldehyde, and water was completely removed from the nanofilter. Before observing the image using the analysis apparatus of the present invention, observation was performed using a general fluorescence microscope to thus confirm that the cells were collected by the nanofilter. Subsequently, in order to calculate the efficiency of collection of the cells by the nanofilter, a cell collection experiment was performed using a control hemocyte sample. A standardized antibody cocktail and a nuclear staining agent (Hoechst 33342, concentration: 5 µg/ml, Ex/Em = 343/483 nm) were added to control hemocytes for quality control (human leukocytes•erythrocyte preservation solution, IMMUNO-TROL Cells, Beckman Coulte Company), and then were reacted at room temperature for 45 minutes in the absence of light. After staining the cells, the concentration of the cell was checked using a cell counter, 200 µl (4500 cells) of the cell suspension prepared by dilution with PBS was added to a new nanofilter, and suction was performed, thus collecting the cells, followed by observation using fluorescence microscopy (FIG. 3A). As a result, the cells were able to be collected with an efficiency of 97 ± 2.2%. From the results, it was found that the analysis apparatus according to the embodiment of the present invention had a sufficient ability of cell collection.

### 7. Verification experiment 2

An experiment on whether CD4 positive T cells were measured using the analysis apparatus according to the embodiment of the present invention was performed. In the experiment, CD4 positive T cells and CD8 positive T cells were used. In order to detect two types of cells using the same excitation light and fluorescence of different wavelengths, dual-color imaging was used, in which staining using two types of fluorescent dye and separation were performed. To this end, a wavelength (λₑₓ = 464.5-499.5 nm, λₑₘ ≥ 519 nm) at which both green fluorescence and yellow fluorescence are simultaneously observed was set as the wavelength passing through the excitation light filter (620). First, an antigen-antibody reaction was performed using a mouse-derived Alexa Fluor 488 (λₑₓ = 499 nm, λₑₘ = 520 nm) labeld anti-human CD4 IgG antibody and a mouse-derived PE-Alexa Fluor 610 (λₑₓ = 567 nm, λₑₘ = 627 nm) labeled anti-human CD8 IgG antibody, so that immunostained CD4 positive T cells and immunostained CD8 positive T cells were collected by the nanofilter. Observation was performed using the analysis apparatus according to the embodiment of the present invention. As a result, it was possible to simultaneously observe fluorescences with which CD4 positive T cells and CD8 positive T cells are labeled using the display means (700) (FIG. 3B). From the results, it was found that the CD4 positive T cells were capable of being measured using the analysis apparatus used in the present invention.

### 8. Verification experiment 3

In order to find the superiority of the measurement of CD4 positive T cells using the analysis apparatus according to the embodiment of the present invention, an experiment was performed using conventional cell group samples.

As a blood sample, control hemocytes (IMMUNO-TROL Cells or IMMUNO-TROL Low Cells, Beckman Coulter) for accuracy control, in which the concentration of T cells in the blood was displayed, were used. On the basis of a value of 350 cells/µl, which is the starting criterion for the use of antiretroviral therapeutic agents, a blood sample having a high CD4 positive T cell count and a CD4/CD8 ratio of more than 1 was defined as a High sample (CD4 positive T cell count: 566 ± 165 cells/µl, CD8 positive T cell count: 263 ± 118 cells/µl, CD4/CD8 = 2.15). A blood sample having a CD4 positive T cell count of less than 350 cells/µl and a CD4/CD8 ratio of less than 1 was defined as a Low sample (CD4 positive T cell count: 140 ± 70 cells/µl, CD8 positive T cell count: 232 ± 116 cells/µl, CD4/CD8 = 0.60). Each blood sample was subjected to an antigen-antibody reaction with each of CD4 positive T cells and CD8 positive T cells for 30 minutes using rat-derived anti-human CD4 IgG and mouse-derived anti-human CD8 IgG mixture solutions (concentration: 10 µg/ml). 200 µl of the blood sample was diluted with 0.1 M PBS so as to contain hemocytes corresponding to 1 µl of blood, thus being set as a sample to be added to the filtration unit (130). Further, the experiment was performed using six Low samples and six High samples.

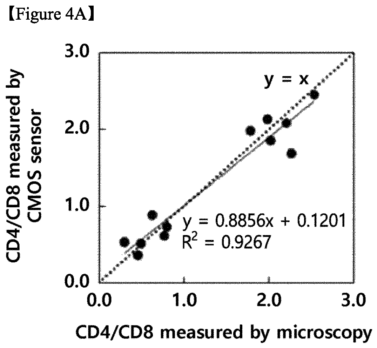

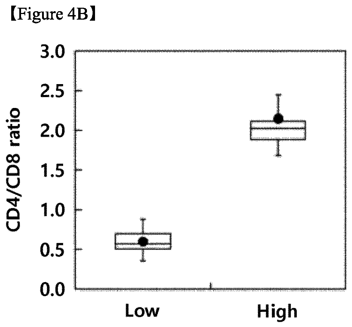

After the cells were collected using the nanofilter, the cells stained so as to have green (CD4 positive T cells) and yellow (CD8 positive T cells) colors were first detected using a fluorescence microscope. Thereafter, the cell count was measured over the entire area of the nanofilter using the analysis apparatus according to the embodiment of the present invention, thus calculating the CD4/CD8 ratio, which was compared to the result of observation using the fluorescence microscope. As a result, a high correlation was obtained therebetween (FIG. 4A). From the result, it was found that the CD4/CD8 ratio could be calculated with the same accuracy as the fluorescence microscope by performing dual-color imaging using the analysis apparatus according to the embodiment of the present invention. Further, as a result of calculation of the CD4/CD8 ratio using the analysis apparatus according to the embodiment of the present invention, a value equivalent to the expected value was obtained (FIG. 4B). From the result, it was verified that High/Low determination of the CD4 positive T cell counts was possible using the analysis apparatus according to the embodiment of the present invention on the basis of the starting criterion for AIDS treatment (CD4 positive T cell count of less than 350 cells/µl or CD4/CD8 < 1).

### 9. Verification experiment 4

The leukocytes (10³ to 10⁴ cells) in 1 µl of the blood sample were collected using the nanofilter according to the embodiment of the present invention, and an experiment was performed using a result value obtained through measurement using the analysis apparatus according to the embodiment of the present invention.

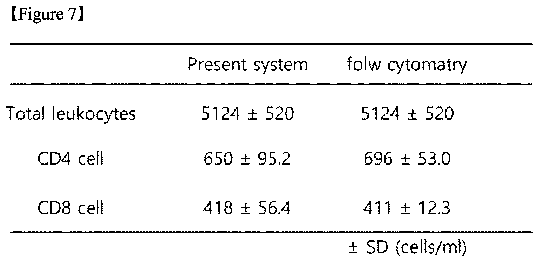

A result value obtained by detection using flow cytometry, which is a conventional method for measuring CD4 positive T cells, was set as a control group. Accordingly, the results shown in FIG. 7 were obtained. From FIG. 7, it can be found that there is no significant difference with the result value of the flow cytometry method, which is the conventional method. The result indicates that the analysis apparatus according to the embodiment of the present invention enables determination of a appropriate starting point for AIDS treatment and effective monitoring after treatment.

Although the present invention has been described with reference to the embodiments shown in the drawings so that those skilled in the art can easily understand and reproduce the invention in the present specification, it will be understood by those skilled in the art that the embodiments are merely for illustrative purposes, and that various modifications and other equivalent embodiments may be made from the embodiments of the present invention. Accordingly, the scope of protection of the present invention should be determined by the claims.

## Claims

1. A method of diagnosing HIV using an HIV diagnostic apparatus comprising a nanofilter, which comprises an upper body (100) for collecting cells, the upper body (100) having a plurality of filtration holes (120) formed through a substrate (110), and in which any one filtration hole of the plurality of filtration holes (120) is adjacent to other filtration holes and an interval between a center of any one filtration hole and centers of the other filtration holes adjacent thereto is 23 µm to 27 µm, the method comprising:
(a) collecting leukocytes among the cells using the nanofilter;
(b) radiating light toward an entire area of the nanofilter using a light source unit (500);
(c) sensing light passing through the nanofilter using a light-sensing unit (600);
(d) outputting an image of the entire area of the nanofilter through a display means (700) based on information obtained by the light-sensing unit (600); and
(e) analyzing the image outputted through the display means (700) using an analyzing means (800),
wherein (e) the analyzing the image comprises analyzing information on a ratio of a CD4 positive T cell count to a CD8 positive T cell count using the analyzing means (800).

2. The method of claim 1, wherein (e) the analyzing the image further comprises:
analyzing information on the CD4 positive T cell count and a ratio of a CD4 positive cell count to a lymphocyte cell count using the analyzing means (800), and
determining a time point at which the information analyzed using the analyzing means (800) indicates that the CD4 positive T cell count is less than 350 cells/µl, or the ratio of the CD4 positive T cell count to the CD8 positive T cell count is less than 1, or the ratio of the CD4 positive T cell count to the lymphocyte cell count is 0.25 or less as a starting time point for AIDS treatment.

3. The method of claim 1, wherein planes of the filtration holes (120) have a circular shape and diameters of the filtration holes (120) are 3 µm to 5 µm.

4. The method of claim 1, wherein the substrate (110) is made up of a metal material or a plastic material.

5. The method of claim 1, wherein the nanofilter further comprises a filtration unit (130) which is positioned on an upper side of the substrate (110) and to which a sample solution is added;
a suction unit (140) which is positioned on a lower side of the substrate (110) and to which a material not filtered through the filtration holes (120) is moved; and
a flow channel (300) through which the suction unit (140) and a lower body (400) are connected to each other, and the method further comprises:
before (a) the collecting the leukocytes,
separating the lower body (400) from the upper body (100) at a constant rate, so that the sample solution added to the filtration unit (130) is sucked into the suction unit (140) at a predetermined rate.

6. The method of claim 5, wherein the predetermined rate is 100 µl/min to 500 µl/min.

7. The method of claim 1, wherein the light-sensing unit (600) comprises:
a lens (610) through which the light passing through the nanofilter is converted to a parallel light form;
an excitation light filter (620) through which light having a specific wavelength among light components passing through the lens (610) passes; and
an image sensor (630) sensing the light passing through the excitation light filter (620).

8. The method of claim 1, further comprising:
after (e) the analyzing the image,
displaying the information analyzed using the analyzing means (800) through the display means (700).

9. The method of claim 1, wherein the cells further comprise JM cells or IMMUNO-TROL cells.

10. The method of claim 1, further comprising:
before (a) the collecting the leukocytes,
reacting a CD4 positive T cell and a CD8 positive T cell with Alexa Fluor 488 marked anti-Human CD4 IgG antibody and PE-Alexa Fluor 610 marked anti-Human CD8 IgG, respectively, for 10 minutes to 30 minutes.
